# EUROPEAN PATENT APPLICATION

(11) **EP 4 748 943 A1**
(43) Date of publication of application: **27.05.2026**
(21) Application number: 25211876.5
(22) Date of filing: 29.10.2025
(51) Int. Cl.: C12Q 1/6837

(54) **CHIP, PREPARATION METHOD THEREFOR AND USE THEREOF**

(30) Priority: 18.11.2024 CN 202411648956
(71) Applicant: GeneMind Biosciences Company Limited, Shenzhen City, Guangdong 518023 (CN)
(72) Inventor: Li, Zhao, Luohu District, Shenzhen City, 518023 (CN); Liu, Lei, Luohu District, Shenzhen City, 518023 (CN); Chen, Fang, Luohu District, Shenzhen City, 518023 (CN)
(74) Representative: Michalski Hüttermann & Partner Patentanwälte mbB

(57) **Abstract**

The present application discloses a method for preparing a spatial chip, a spatial chip, and a use thereof. The method comprises: hybridizing to attach a single-stranded nucleic acid molecule to a surface of a solid-phase substrate, the single-stranded nucleic acid molecule comprising a first fixed sequence, a spatial barcode sequence, a sequencing primer binding sequence and a second fixed sequence, a first probe being attached to the surface of the solid-phase substrate of the chip, and the first probe comprising a complementary sequence of the first fixed sequence and a complementary sequence of the second fixed sequence; ring closing, causing the single-stranded nucleic acid molecule to form a circular nucleic acid molecule; rolling circle amplification; sequencing, to obtain sequence information of the spatial barcode sequence; synthesizing a single-stranded nucleic acid molecule by using the rolling circle amplification product as a template, to obtain a single-stranded nucleic acid molecule library; and fixing and clustering, fixing the single-stranded nucleic acid molecule library on the surface of the solid-phase substrate to form a single-stranded nucleic acid molecule cluster. According to the present application, a chip having a nucleic acid probe carrying spatial position coordinate information can be prepared.

## Description

### TECHNICAL FIELD

The present application relates to the field of biotechnology, and in particular, to a chip, a preparation method therefor, and a use thereof.

### BACKGROUND ART

At present, in biochips for next-generation sequencing, signal amplification is mostly achieved by means of performing parallel sequencing on nucleic acid templates in amplification clusters formed by surface amplification or DNB nanoballs formed by rolling circle amplification. However, there is limited room for an increase in the copy number obtained by these two amplification methods. In the case of using a biochip for spatial omics analysis, it is desirable to have a spatial chip that can obtain a higher copy number, so as to satisfy spatial omics research based on samples including cells and tissues, and having relatively complex biological information. To this end, we have developed a method for forming amplification clusters different from probe clusters or DNB nanoballs, to be used for preparing a biochip that enables an increase in copy number.

### SUMMARY OF THE INVENTION

Objects of the present application are to provide a chip, a preparation method therefor and a use thereof, particularly a spatial chip preparation method, a spatial chip and a use thereof, aiming to increase the copy number of nucleic acid templates on a surface of the chip, such that the biochip has better application prospects in the field of spatial omics, which has higher requirements for the density of nucleic acid molecules.

The present application adopts the following technical solutions:
A first aspect of the present application discloses a method for preparing a spatial chip, comprising: hybridizing to attach a single-stranded nucleic acid molecule to a surface of a solid-phase substrate, wherein the single-stranded nucleic acid molecule comprises a first fixed sequence, a spatial barcode sequence, a sequencing primer binding sequence and a second fixed sequence, the first fixed sequence and the second fixed sequence being located at two ends of the single-stranded nucleic acid molecule respectively, a first probe is attached to the surface of the solid-phase substrate, and the first probe comprises a complementary sequence of the first fixed sequence and a complementary sequence of the second fixed sequence; ring closing, comprising: linking the first fixed sequence and the second fixed sequence by using a ligase, such that the single-stranded nucleic acid molecule forms a circular nucleic acid molecule; rolling circle amplification, comprising: subjecting the circular nucleic acid molecule to rolling circle amplification by using the complementary sequence of the second fixed sequence as a primer; sequencing, comprising: sequencing the rolling circle amplification product by using a sequencing primer to obtain sequence information of the spatial barcode sequence; synthesizing a single-stranded nucleic acid molecule library, comprising: synthesizing a single-stranded nucleic acid molecule by using the rolling circle amplification product as a template, to obtain the single-stranded nucleic acid molecule library; and fixing and clustering, comprising: fixing the single-stranded nucleic acid molecule library onto the surface of the solid-phase substrate to form a single-stranded nucleic acid molecule cluster.

In one implementation of the present application, the single-stranded nucleic acid molecule comprises, sequentially from the 5' end to the 3' end, the first fixed sequence, the spatial barcode sequence, the sequencing primer binding sequence and the second fixed sequence, the first probe sequentially comprises the complementary sequence of the second fixed sequence and the complementary sequence of the first fixed sequence, and the probe is attached to the surface of the solid-phase substrate by means of the complementary sequence of the first fixed sequence.

In one implementation of the present application, the first probe is provided with a first cleavable site.

In one implementation of the present application, the first cleavable site is at least one of a photocleavable site, a chemically cleavable site, and an enzyme cleavage site.

In one implementation of the present application, after the step of fixing and clustering, the rolling circle amplification product and the first probe are cleaved from the surface of the solid-phase substrate by means of the first cleavable site.

In one implementation of the present application, the spatial barcode sequence is formed by polymerization of a number n of nucleotides, and the number of the single-stranded nucleic acid molecule clusters on the surface of the solid-phase substrate is less than or equal to 4ⁿ.

In one implementation of the present application, in the step of synthesizing the single-stranded nucleic acid molecule library, a sequence in which a second cleavable site is provided is used as a synthesis primer for synthesizing the single-stranded nucleic acid molecule.

In one implementation of the present application, the second cleavable site is at least one of a photocleavable site, a chemically cleavable site, and an enzyme cleavage site.

In one implementation of the present application, the first probe is provided with the first cleavable site, and the first cleavable site and the second cleavable site are not completely identical.

In one implementation of the present application, the 5' end of the synthesis primer is provided with a coupling group, the coupling group being used for binding to the surface of the solid-phase substrate; and/or, the surface of the solid-phase substrate is linked with a second probe, and the 5' end of the synthesis primer is provided with a complementary sequence of the second probe.

In one implementation of the present application, the complementary sequence of the second probe is polyN, the N being selected from one of A or a derivative thereof, T/U or a derivative thereof, G or a derivative thereof, and C or a derivative thereof.

In one implementation of the present application, after the step of fixing and clustering, the single-stranded nucleic acid molecule library is linked with a capture sequence, the capture sequence being used for capturing a biomolecule to be detected.

In one implementation of the present application, the capture sequence comprises polyT.

In one implementation of the present application, the single-stranded nucleic acid molecule library is linked with the capture sequence and a UMI sequence, the UMI sequence being a random base sequence.

A second aspect of the present application discloses a spatial chip prepared by the preparation method disclosed in the first aspect.

In one implementation of the present application, the single-stranded nucleic acid molecule cluster has a cross-sectional diameter of 0.5 µm to 1 µm, and the copy number of the single-stranded nucleic acid molecule in the single-stranded nucleic acid molecule cluster is greater than 30,000.

A third aspect of the present application discloses a use of the spatial chip disclosed in the second aspect in obtaining spatial distribution information of biomolecules.

In one implementation of the present application, the use includes spatial transcriptomic analysis, spatial proteomic analysis, and single cell spatial analysis.

The beneficial effect of the present application is as follows:
The present application adopts a clustering method different from probe clusters or DNB nanoballs to prepare a chip having nucleic acid probes that carry spatial position coordinate information. The method can increase the copy number of nucleic acid molecules on the surface of the chip.

### BRIEF DESCRIPTION OF THE DRAWINGS

FIG. 1 is a schematic structural diagram of a spatial chip according to the present application;
FIG. 2 is a schematic diagram of a method for preparing a spatial chip according to the present application;
FIG. 3 is a schematic diagram of rendering a spatial chip to be linked with a capture sequence according to the present application;
FIG. 4 shows a schematic diagram of a spatial reverse transcription sequencing method according to the present application;
FIG. 5 is a diagram of the sequencing Q30 result of performing sequencing on a rolling circle amplification product according to examples of the present application;
FIG. 6 is a diagram of the base distribution result of sequencing a rolling circle amplification product according to examples of the present application;
FIG. 7 is a diagram of the quality control result of the quality control performed on a spatial chip according to examples of the present application;
FIG. 8 is a diagram of the result of identification of cDNA products according to examples of the present application; and
FIG. 9 is a diagram of the result of restoring spatial positional information by plotting compared data against bin50µm according to examples of the present application.

### DESCRIPTION OF THE PREFERRED EMBODIMENTS

The present invention will be further described in detail below with reference to specific embodiments in conjunction with the accompanying drawings. In the following embodiments, many details are described to make the present application better understood. However, those skilled in the art can realize, without expending effort, that some of the features may, depending on different cases, be omitted, or replaced by other methods or steps. In some cases, some operations related to the present application are not shown or described in the description, so as to avoid the core part of the present application being overwhelmed by excessive description. It is not necessary to describe these related operations in detail for those skilled in the art, who can completely understand the related operations in view of the description in the specification, as well as general technical knowledge in the art.

In addition, the characteristics, operations, or features described in the specification may be combined in any suitable manner to form various embodiments. In addition, the steps or actions in the description of a method may also be exchanged or adjusted in sequence, in a manner that is obvious to those skilled in the art. Therefore, the various sequences in the specification and the accompanying drawings are merely for the purpose of clearly describing a certain embodiment, and are not meant to be a necessary sequence, unless it is otherwise stated that a certain sequence must be followed.

The serial numbers themselves, such as "first" and "second", for coding objects herein are only used to distinguish the described objects, and to distinguish objects such as substances, orientations, interfaces, messages, requests, and terminals from each other, and cannot be understood as indicating or implying the relative importance of the objects or implicitly specifying the number of the indicated objects. For example, without departing from the scope of the embodiments of the present disclosure, the "first fixed sequence" may also be referred to as the "second fixed sequence", and conversely, the "second fixed sequence" may be referred to as the "first fixed sequence". Thus, features defined by "first" and "second" may explicitly or implicitly include one or more of the features.

The term "chip" as used herein refers to a solid-phase substrate having a surface, e.g. a planar surface, at least one surface of which has a biomolecule to be detected, , e.g. a nucleic acid molecule, attached thereto, or has a biomolecule used for binding to a biological sample to be detected, e.g. a nucleic acid molecule, attached thereto. In sequencing and sequencing-related applications, a chip is also referred to as a biochip, a sequencing chip, or a flow cell.

The term "library" as used herein is a pool/collection of nucleic acid molecules comprising a plurality of target fragments. Typically, the target fragments/fragments to be detected are processed, e.g., by adding a known sequence to one or both ends of the target fragments, e.g., by adding a linker (sequencing linker), such that the library can be linked or fixed onto a chip, so as to be suitable for being loaded onto a sequencing platform for sequencing.

The term "sequencing" used herein may also be referred to as "nucleic acid sequencing" or "gene sequencing". The three terms are semantically interchangeable, and all refer to determining the type and arrangement order of bases or nucleotides (including nucleotide analogs) in a nucleic acid molecule. Said sequencing comprises a process of binding nucleotides to a template and collecting the corresponding signals emitted from the nucleotides (including analogs). Said sequencing includes sequencing by synthesis (SBS) and/or sequencing by ligation (SBL), includes DNA sequencing and/or RNA sequencing, and includes long fragment sequencing and/or short fragment sequencing; said long fragment and short fragment are in relative terms, e.g., nucleic acid molecules longer than 1 Kb, 2 Kb, 5 Kb or 10 Kb may be referred to as long fragments, and nucleic acid molecules shorter than 1 Kb or 800 bp may be referred to as short fragments.

Sequencing generally comprises multiple cycles of a process, to achieve determination of the types and arrangement order of multiple bases or nucleotides on a nucleic acid template. In examples of the present application, each cycle of the "process to achieve determination of the types and arrangement order of multiple bases or nucleotides on a nucleic acid template" is referred to as "one cycle of sequencing". A "cycle", also referred to as a "sequencing cycle", can be defined as one instance of base extension of the four kinds of nucleotide/base. In other words, a "sequencing cycle" can be defined as completing the determination of the type of the base or nucleotide at any designated position on the template. For a sequencing platform that achieves sequencing based on a polymerization or ligation reaction, one cycle of sequencing comprises a process of achieving binding of the four kinds of nucleotide (including nucleotide analogs) to the so-called nucleic acid template in a base-complementary manner at one time, and collecting the corresponding signals that are emitted. For a platform that achieves sequencing based on a polymerization reaction, the reaction system comprises nucleotides as reaction substrates, a polymerase, and a nucleic acid template onto which a segment of sequence (sequencing primer) is bound. On the basis of the principle of base pairing and the mechanism of the polymerization reaction, an added nucleotide as a reaction substrate, catalyzed by the polymerase, is linked to the sequencing primer to achieve binding of the nucleotide to a particular position of the nucleic acid template. Generally, one cycle of sequencing may comprise one instance or multiple instances of base extension (repeat). For example, the four kinds of nucleotide are sequentially added to a reaction system, and base extension and collection of a corresponding reaction signal are performed for each; one cycle of sequencing comprises four instances of base extension. Alternatively, for example, any combination of the four kinds of nucleotide is added to a reaction system. For example, the four kinds of nucleotide are added in sets of two and two, or sets of one and three, and base extension and collection of corresponding reaction signals are performed for the two sets respectively; one cycle of sequencing comprises two instances of base extension. As another example, the four kinds of nucleotide are added to a reaction system at the same time for base extension and collection of reaction signals; one cycle of sequencing comprises one instance of base extension.

In each cycle of sequencing, each identified base may be scored by using a quality value for evaluating the accuracy of the identification result. The quality value may be a quality score (typically represented by a Q value or a Qphred), which reflects the reliability and error rate (e) of base calling in a sequencing process. The method for calculating the Q value is as follows: Q = -10log₁₀e. It can be seen from this formula that the larger the Q value, the lower the possibility of calling errors, and the higher the reliability. The Q value, which is generally used for statistics, corresponds to different error rates, such as Q20, Q30, Q40, etc. In particular, Q20 corresponds to a correct calling rate of 99%, and an incorrect calling rate of 1%; Q30 corresponds to a correct calling rate of 99.9%, and an incorrect calling rate of 0.1%; Q40 corresponds to a correct calling rate of 99.99%, and an incorrect calling rate of 0.01%; and so on and so forth.

The term "nucleic acid molecule" as used herein means a polymeric form of nucleotides of any length, and may include ribonucleotides or analogs thereof, deoxyribonucleotides or analogs thereof, and mixtures formed of said nucleotides or analogs thereof. Said nucleic acid molecule may refer to a single-stranded polynucleotide or a double-stranded polynucleotide. The nucleotides in a nucleic acid molecule may include naturally occurring nucleotides and functionally alternative analogs thereof. Examples of analogs are capable of hybridizing to a nucleic acid in a sequence-specific manner, or are capable of serving as a template for the replication of a particular nucleotide sequence. A naturally occurring nucleotide typically has a backbone comprising a phosphodiester bond. The structure of an analog may have a backbone linkage that includes any type of substitution known in the art. A naturally occurring nucleotide typically has a deoxyribose sugar (e.g., found in DNA) or a ribose sugar (e.g., found in RNA). The structure of an analog may have an alternative sugar moiety, including any type known in the art. A nucleotide may comprise a natural base or non-natural base. The bases in natural DNA may include one or more of adenine, thymine, cytosine, and/or guanine, and the bases of natural RNA may include one or more of adenine, uracil, cytosine, and/or guanine. A nucleotide can also use any non-natural base or base analog, such as a locked nucleic acid (LNA) and a bridged nucleic acid (BNA).

The term "nucleic acid template" as used herein refers to a master nucleic acid molecule or a fragment of the master nucleic acid molecule to which nucleotides or nucleotide analogs are bound through successive cycles of base extension. The so-called "nucleic acid template" may be the entire sequence of a nucleic acid molecule or a partial fragment of the nucleic acid molecule. The "nucleic acid template" referred to may be a nucleic acid fragment used as a template in an extension reaction in which sequencing by synthesis is performed. Since the bases added by the extension reaction and the sequencing template satisfy the base pairing principle, the sequence of the sequencing template can be determined by determining the type of base added in each cycle of extension reaction. The sequence of the nucleic acid template herein may generally include, but is not limited to, at least one of a target molecule sequence, a UMI sequence, and a sample tag sequence, sometimes also referred to herein as an "insert" or "target molecule".

The term "cluster" as used herein refers to a population of nucleic acid molecules attached to a surface of a solid-phase base board to form a feature or site. Generally speaking, the nucleic acid molecules in a population of nucleic acid molecules are typically nucleic acid molecules of identical sequence, forming a monoclonal cluster by means of amplification or cloning. For example, an amplification cluster formed by means of amplifying the same nucleic acid molecule by bridge PCR, rolling circle amplification or other means of amplification, and comprising multiple copies of the nucleic acid molecule.

The term "primer", also known as a "probe", as used herein refers to an oligonucleotide or nucleic acid molecule that can be hybridized to a target sequence of interest. In examples, a primer functions as a substrate onto which nucleotides can be polymerized by a polymerase. For example, the primer may serve as a starting point for DNA or RNA synthesis. For example, a sequencing primer can hybridize to a synthetic nucleic acid template strand so as to prime the synthesis of a new strand that is complementary to the synthetic nucleic acid template strand. The primer may comprise any combination of nucleotides or analogs thereof. In some examples, the primer is a single-stranded oligonucleotide or polynucleotide.

The terms "linked", "fixed" and the like as used herein are to be understood broadly, and refer to a state in which two objects are joined, fastened, adhered, attached or bound to one another. For example, they may be fixedly linked or reversibly linked, may be linked directly or linked via an intermediate medium, may be linked via a chemical bond, such as covalently linked, may be chemically or physically adsorbed, etc. For example, a nucleic acid molecule may be attached to a material such as a gel or a solid support by means of a covalent or non-covalent bond. A covalent bond is characterized by sharing of an electron pair between atoms; a non-covalent bond is a chemical bond that does not involve sharing of an electron pair, and may include, for example, a hydrogen bond, an ionic bond, van der Waals force, a hydrophilic interaction and a hydrophobic interaction.

The term "cleavage site" as used herein refers to a location in a nucleic acid molecule that is susceptible to bond cleavage. The position may be specific for a particular chemical, enzymatic, or physical process that results in bond cleavage. For example, the position may be an abasic nucleotide, or a nucleotide having a base that is susceptible to being removed to create an abasic site. Examples of nucleotides which are susceptible to the removal include uracil and 8-oxo-guanine. The position may also be at or near a recognition sequence for a restriction endonuclease, such as a nicking enzyme.

The term "hybridization", also known as nucleic acid hybridization, as used herein refers to a process in which two polynucleotides (DNA-DNA, DNA-RNA, RNA-RNA, etc.) that are completely or partially complementary in sequence form a non-covalent bond by means of Watson-Crick base pairing, thereby forming a stable homologous or heterologous double-stranded nucleic acid molecule.

As used herein, the terms "a", "an" and "the" may include plural references unless the context clearly indicates otherwise. Throughout the specification, for example, "one example", "an example", "another example", "a particular example", "a related example", "a certain example", "an additional example", "a further example", or a combination thereof means that particular features, structures, or characteristics described in connection with the example are included in at least one example of the present disclosure. Thus, the foregoing phrases appearing in various places throughout this specification are not necessarily all referring to the same example. In addition, the particular features, structures or characteristics may be combined in one or more examples in any suitable manner.

Sequencing can be performed on a sequencing platform, which may be selected from, but is not limited to, Hiseq/Miseq/Nextseq/Novaseq sequencing platforms from Illumina, the Ion Torrent platform from Thermo Fisher/Life Technologies, BGISEQ and MGISEQ platforms from BGI, and single-molecule sequencing platforms. Either single-end sequencing or double-end sequencing may be selected as the manner of sequencing. The obtained sequencing results/data, i.e., the fragments read out by the sequencing, are referred to as reads. The length of a read is referred to as read length.

In the description of the present application, the concentrations mentioned for relevant components may not only refer to the specific contents of the components, but also represent proportional relationships among the contents of the components. Therefore, either scaling up or scaling down according to the contents of the relevant components in the examples of the description of the present application would be within the scope disclosed in the examples of the description of the present application.

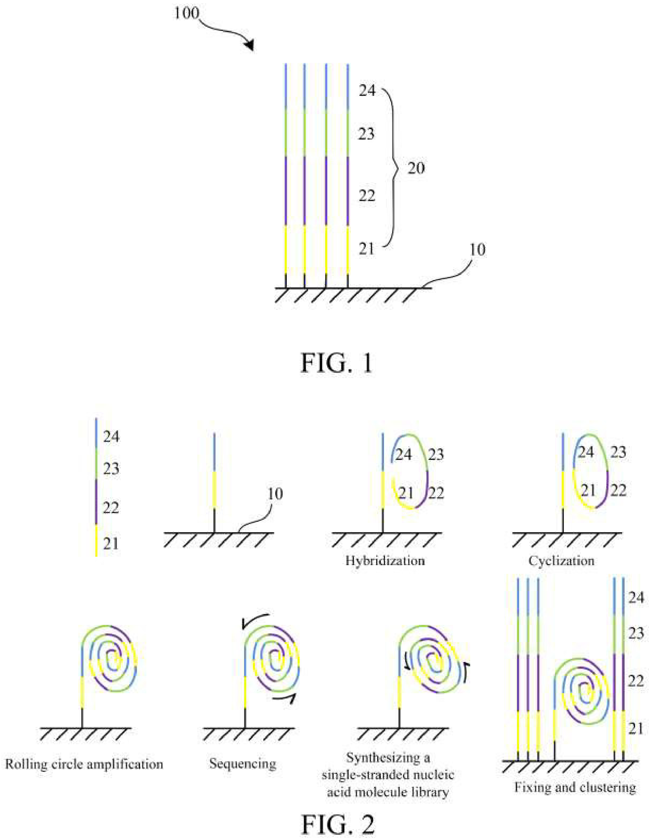

Referring to FIG. 1, an aspect of the present application provides a spatial chip 100.

As shown in FIG. 1, the spatial chip 100 comprises a solid-phase substrate 10. The solid-phase substrate 10 is used to support nucleic acid molecules and, at the same time, provide a reaction site for the identification that occurs based on nucleic acid molecules, the detection of the characteristics of nucleic acid molecules, and other operations that occur on nucleic acid molecules.

The solid-phase substrate 10 is a solid-phase base board. The material thereof may be selected from materials such as quartz, glass, crystal, silicon chip, silicon nitride, silicon dioxide, etc., and may also be plastic, ceramic, PET (polyethylene terephthalate), PMMA (polymethyl methacrylate), hydrogel, metal, or any other suitable material. The shape of the solid-phase substrate 10 may be flexibly configured, or the solid-phase substrate 10 may have a special shape depending on the material of the substrate 10. In one embodiment, the solid-phase substrate 10 is planar. In another embodiment, the solid-phase substrate 10 may be non-planar. By way of illustration, the solid-phase substrate 10 is a microbead or magnetic bead, and in this case, the solid-phase substrate 10 has a curved surface, such as a spherical surface or a hemispherical surface. Of course, on the basis of the described two examples, a surface of the solid-phase substrate 10 may be modified so as to form a modification comprising, e.g., a dendrimer on the surface thereof. In this case, the surface may be an irregular surface having undulations or unevenness.

When the solid-phase substrate 10 is planar, in some examples, the overall shape of the solid-phase substrate 10 may be a sheet structure of rectangular, square, rhombic, otherwise polygonal, circular, elliptical, or irregular shape. In some examples, in order to match the structure in a fixing apparatus and/or instrument of most of the currently available spatial chips for accommodating and fixing the solid-phase substrate 10, the overall shape of the solid-phase substrate 10 is configured to be a rectangular sheet structure. In addition, in some embodiments, after a chip for sequencing is subjected to sequencing as a whole, the chip is cut, so as to select a sequenced region to form the solid-phase substrate 10. One chip can be cut into a plurality of solid-phase substrates 10. Hence, array chips having known information (including barcode sequences bound to the solid-phase substrate 10 and the physical coordinates of each barcode sequence) can be prepared in advance and in batches, thereby improving the preparation efficiency of the spatial chip. It should be understood that the solid-phase substrate 10 provided in examples of the present application may be used as a carrier for sequencing, and may also be used as a carrier for other nucleic acid detections. By way of illustration, the solid-phase substrate 10 is not strictly limited in terms of the source and the chip characteristics, and includes, but is not limited to, the sequencing chips provided by sequencing platforms such as Illumina, Genemind and MGI, sequencing microbeads provided by platforms such as ThermoFisher and Ultima, and gene chips provided by Affymetrix etc., among other like or similar chip base boards.

In examples of the present application, when the solid-phase substrate 10 is overall planar, the solid-phase substrate 10 may be formed having micro-nano pores, which may also be understood as recesses; alternatively, a surface of the chip base board may be formed having micro-nano columns, which may also be understood as protrusions. The cross-sectional shape of the micro-nano pores and micro-nano columns is not strictly limited, and may be circular, square, rectangular, rhombic, elliptical, otherwise polygonal, in particular equilateral polygonal, or another irregular shape. The micro-nano pores refer to holes or recesses having a pore size at a micron or nanometer scale, and the micro-nano columns refer to columnar protrusions having a maximum cross-sectional radial dimension at a micron or nanometer scale. In some examples, the micro-nano pores or micro-nano columns are distributed in an array on the surface of the solid-phase substrate 10.

In one embodiment, a material having a light transmittance of greater than or equal to 90% is selected for the solid-phase substrate 10. Thus, a reaction or operation on the surface of the solid-phase substrate 10 can be detected by means of currently existing sequencing methods based on optical signal detection. Theoretically, both surfaces of the solid-phase substrate 10 can be used as detection surfaces or monitoring surfaces for optical signals, which expands the application of the spatial chip.

In some examples, the solid-phase substrate 10 has a thickness of 0.05 mm to 0.3 mm. By way of illustration, the solid-phase substrate 10 specifically has a thickness of 0.05 mm, 0.10 mm, 0.15 mm, 0.20 mm, 0.25 mm, 0.30 mm, etc. Hence, the thickness of the solid-phase substrate 10 being within this range is, in one aspect, beneficial to maintaining the structural integrity and informational integrity of the solid-phase substrate 10 when the solid-phase substrate 10 is separated from the chip mother substrate; in another aspect, the thickness of the solid-phase substrate 10 being within this range is beneficial for an optical system to image optical signals generated on the surface of the solid-phase substrate 10, so as to obtain an optical image.

In examples of the present application, at least one surface of the solid-phase substrate 10 has a plurality of separate single-stranded nucleic acid amplification clusters 20. The single-stranded nucleic acid amplification clusters 20 may be randomly distributed on the surface of the solid-phase substrate 10, or may be distributed on the surface of the chip 11 in the form of an array. In some embodiments, the single-stranded nucleic acid amplification clusters 20 are bound to a modified surface of the solid-phase substrate 10. In some other embodiments, the single-stranded nucleic acid amplification clusters 20 may be bound to a surface of the micro-nano pores or micro-nano columns of the solid-phase substrate 10. That is, the solid-phase substrate 10 is a nanowell or nanopore chip, and the single-stranded nucleic acid amplification clusters 11 are fixed in the nanowells or nanopores of the solid-phase substrate 10.

In certain embodiments, the single-stranded nucleic acid amplification clusters 20 are formed in micro-nano pore structures of the solid-phase substrate 10. In some examples, the micro-nano pore structures have a depth of 10 nm to 100 µm, so as to meet the space required for sample binding or reaction. In some examples, the depth of the micro-nano pore structures may be 100 nm to 1 µm, or may be 200 nm to 800 nm. In some embodiments, the micro-nano pore structures are circular pore structures, and in such embodiments, the micro-nano pore structures have a diameter of 10 nm to 100 µm, so as to meet the space required for sample binding or reaction. In some examples, the diameter of the micro-nano pore structures is 50 nm to 10 µm, or may be 100 nm to 1 µm, or 200 nm to 800 nm.

In some embodiments, the distribution of the single-stranded nucleic acid amplification clusters 20 on the solid-phase substrate 10 may be random. That is, the single-stranded nucleic acid amplification clusters 20 are randomly distributed on the solid-phase substrate 10. As a possible implementation, the single-stranded nucleic acid amplification clusters 20 are arranged in an array on the solid-phase substrate 10, such that the reaction signals generated from the detection reactions that the regularly arranged single-stranded nucleic acid amplification clusters 20 undergo have better identifiability. By way of illustration, when the detection reactions on the surface of the single-stranded nucleic acid amplification clusters 20 generate optical signals, the regularly-distributed optical signals are beneficial for data analysis or algorithm statistics, thereby facilitating the acquisition of the corresponding detection parameters or detection results; or, based on the array optical signals obtained at the single-stranded nucleic acid amplification clusters 20, the accuracy and detection efficiency of the detection and analysis results can be improved.

The single-stranded nucleic acid amplification clusters 20 comprise a plurality of single-stranded nucleic acid molecules having the same nucleotide sequence. In one embodiment, the single-stranded nucleic acid molecules comprise a first fixed sequence 21, a spatial barcode sequence 22, a sequencing primer binding sequence 23, and a second fixed sequence 24.

In the spatial chip 100 provided in examples of the present application, each of the single-stranded nucleic acid amplification clusters 20 has a unique spatial barcode sequence 22, such that when the spatial chip 100 is used for detection of a biological sample, the spatial information of the biological sample can be obtained by analyzing the barcode sequence 22 and its positional information.

In certain embodiments, the spatial barcode sequence 22 is formed by polymerization of a number n of nucleotides, and the number of the single-stranded nucleic acid molecule clusters on the surface of the solid-phase substrate 10 is less than or equal to 4ⁿ (4 to the power of n). In some examples, the number of the single-stranded nucleic acid amplification clusters 20 on the solid-phase substrate 10 is m, and the number of nucleotides constituting the spatial barcode sequence 22 is n, and n and m satisfy: 4ⁿ ≧ m. Thus, 4ⁿ different spatial barcode sequences 22 are obtained by the arrangement and combination of the four nucleotides A, T or U, G, and C, such that each of the single-stranded nucleic acid amplification clusters 20 on the surface of the solid-phase substrate 10 has a spatial barcode sequence 22 having a unique sequence.

In some examples, n is a natural number greater than or equal to 28, and in this case, at least 4²⁸ barcodes of different sequences can be formed by 28 or more nucleotides, meeting the requirement for the number of the single-stranded nucleic acid amplification clusters 20 on a surface of the solid-phase substrate 10 with an area of no more than 1,000 mm².

In examples of the present application, the density of the amplification clusters on the solid-phase substrate 10 may be set based on the complexity of the biological sample to be detected, so as to obtain a better balance between the throughput of the detection and the accuracy of the detection result. In some embodiments, the area of a single-stranded nucleic acid amplification cluster 20 in the solid-phase substrate 10 is 0.25 µm² to 100 µm². By way of illustration, the area of a single-stranded nucleic acid amplification cluster 20 in the solid-phase substrate 10 may be 0.25 µm², 0.5 µm², 0.8 µm², 1.0 µm², 5.0 µm², 10.0 µm², 15.0 µm², 20.0 µm², 25.0 µm², 30.0 µm², 35.0 µm², 40.0 µm², 45.0 µm², 50.0 µm², 55.0 µm², 60.0 µm², 65.0 µm², 70.0 µm², 75.0 µm², 80.0 µm², 85.0 µm², 90.0 µm², 95.0 µm², 100 µm², etc.

In some embodiments, the distance between adjacent single-stranded nucleic acid amplification clusters 20 is 10 nm to 100 µm. It should be understood that when the single-stranded nucleic acid amplification clusters 20 are of a regular shape, such as a circular, elliptical, square, rectangular, or otherwise equilateral polygonal shape, the distance between adjacent single-stranded nucleic acid amplification clusters 20 refers to the center-to-center distance between adjacent single-stranded nucleic acid amplification clusters 20; when the single-stranded nucleic acid amplification clusters 20 are of an irregular shape, the distance between adjacent single-stranded nucleic acid amplification clusters 20 is the minimum distance between the adjacent single-stranded nucleic acid amplification clusters 20, which refers to the shortest connection line among all connection lines between any point on an outer edge of a single-stranded nucleic acid amplification cluster 20 and any point on an outer edge of an adjacent single-stranded nucleic acid amplification cluster 20. In some examples, the distance between adjacent single-stranded nucleic acid amplification clusters 20 may be in the range of from 50 nm to 10 µm, or may be in the range of from 100 nm to 1 µm, or may be in the range of from 200 nm to 800 nm, and of course, may be in the range of any interval within 10 nm to 100 µm. For the single-stranded nucleic acid amplification clusters 20 in examples of the present application, the distances between adjacent single-stranded nucleic acid amplification clusters 20 may, within the foregoing ranges, be completely the same or different.

In some examples, the single-stranded nucleic acid amplification clusters 20 are arranged in an array on the surface of the solid-phase substrate 10, and the distances between any two adjacent single-stranded nucleic acid amplification clusters 20 are the same. By way of illustration, the distance between adjacent single-stranded nucleic acid amplification clusters 20 may be 0.1 µm, 0.2 µm, 0.3 µm, 0.4 µm, 0.5 µm, 0.6 µm, 0.7 µm, 0.8 µm, 0.9 µm, 1.0 µm, 1.2 µm, 1.5 µm, 1.8 µm, 2.0 µm, 2.2 µm, 2.5 µm, 2.8 µm, 3.0 µm, 3.2 µm, 3.5 µm, 3.8 µm, 4.0 µm, 4.2 µm, 4.5 µm, 4.8 µm, 5.0 µm, 5.2 µm, 5.5 µm, 5.8 µm, 6.0 µm, 6.2 µm, 6.5 µm, 6.8 µm, 7.0 µm, 7.2 µm, 7.5 µm, 7.8 µm, 8.0 µm, 8.2 µm, 8.5 µm, 8.8 µm, 9.0 µm, 9.2 µm, 9.5 µm, 9.8 µm, 10.0 µm, etc.

In examples of the present application, the density of the single-stranded nucleic acid amplification clusters 20 on the solid-phase substrate 10 can be set according to requirements such as the complexity of the biological sample to be detected. In some embodiments, the solid-phase substrate 10 may be subjected to surface treatment, such that a plurality of separate positions having been subjected to surface treatment are capable of binding to single-stranded nucleic acid molecules, and surfaces of regions other than the plurality of separate positions are not capable of binding to single-stranded nucleic acid molecules, thereby determining the region and density of distribution of the single-stranded nucleic acid amplification clusters 20 so as to define the position and density of the single-stranded nucleic acid amplification clusters 20 on the solid-phase substrate 10. The manner of surface treatment and the result of surface treatment are not limited, so long as the position and density of the single-stranded nucleic acid amplification clusters 20 on the solid-phase substrate 10 can be defined. By way of illustration, a reactive group that is capable of binding to the single-stranded nucleic acid molecules may be formed at some of the separate positions, or a probe that can be complementary to a portion of the sequence of the single-stranded nucleic acid molecules may be attached at some of the separate positions. In some examples, the single-stranded nucleic acid amplification clusters 20 are arranged on the solid-phase substrate 10 at a density of 5 to 800 clusters/mm². By way of illustration, the density of the single-stranded nucleic acid amplification clusters 20 arranged on the solid-phase substrate 10 may be 5 to 500 clusters/mm², 50 to 500 clusters/mm², 100 to 500 clusters/mm², 100 to 600 clusters/mm², 100 to 700 clusters/mm², 100 to 800 clusters/mm², 200 to 500 clusters/mm², 200 to 600 clusters/mm², 200 to 700 clusters/mm², 200 to 800 clusters/mm², 300 to 500 clusters/mm², 300 to 600 clusters/mm², 300 to 700 clusters/mm², 300 to 800 clusters/mm², 400 to 500 clusters/mm², 400 to 600 clusters/mm², 400 to 700 clusters/mm², 400 to 800 clusters/mm², 500 to 600 clusters/mm², 500 to 700 clusters/mm², 500 to 800 clusters/mm², 600 to 700 clusters/mm², 600 to 800 clusters/mm², 700 to 800 clusters/mm², etc.

In some examples, a single-stranded nucleic acid molecule cluster 20 in the solid-phase substrate 10 has a cross-sectional diameter of 0.5 µm to 1 µm, and the copy number of the single-stranded nucleic acid molecules in the single-stranded nucleic acid molecule cluster 20 is greater than 30,000. By way of illustration, in an area of 0.5 µm to 1 µm, the copy number of the single-stranded nucleic acid molecule is 30,000 to 35,000, 35,000 to 40,000, 40,000 to 50,000, 50,000 to 60,000, 60,000 to 70,000, 70,000 to 80,000, 80,000 to 90,000, 90,000 to 100,000, 100,000 to 110,000, 110,000 to 180,000, etc., and thus, after the single-stranded nucleic acid amplification clusters 20 of the solid-phase substrate 10 are released, the density thereof could reach 10 nmol/L or higher.

In examples of the present application, since the spatial barcode sequences 22 of different single-stranded nucleic acid amplification clusters 20 are different from each other, the spatial barcode sequences 22 can be sequenced, and the physical coordinates of the respective single-stranded nucleic acid amplification clusters 20 in the spatial chip 100 can be obtained by means of the sequencing results. The physical coordinates set forth in examples of the present application refer to the positional information of the single-stranded nucleic acid amplification clusters 20 on the surface of the chip 100, specifically the solid-phase substrate 10, and may be represented by coordinate information such as (x, y). Thus, the position of each of the single-stranded nucleic acid amplification clusters 20 in the solid-phase substrate 10 has a one to-one correspondence with the spatial barcode sequence 22. Therefore, after acquiring signals obtained by a detection reaction, the spatial barcode sequence 22 of the single-stranded nucleic acid amplification cluster 20 can be obtained based on the physical coordinates of the single-stranded nucleic acid amplification cluster 20. Further, other information such as sequence information of the polynucleotide chain linkage where the spatial barcode sequence 22 is located can be obtained, and this information can be used as the basis of detection and analysis to realize the analysis and result determination of the detection reaction.

In examples of the present application, the sequencing primer binding sequence 23 is used to sequence the spatial barcode sequence 22.

In one embodiment, the first fixed sequence 21, the spatial barcode sequence 22, the sequencing primer binding sequence 23 and the second fixed sequence 24 are sequentially linked, and the first fixed sequence 21 is located adjacent to the solid-phase substrate 10. In some examples, the first fixed sequence 21 is attached to the solid-phase substrate 10. In some examples, the first fixed sequence 21 is attached to the solid-phase substrate 10 by means of chemical bonding, physical adsorption, electrostatic adsorption, or van der Waals force.

In one implementation, the solid-phase substrate 10 is bound with a compound, and the first fixed sequence 21 is attached to the solid-phase substrate 10 through the compound. The first fixed sequence 21 is bound to the solid-phase substrate 10 by being linked to a compound, in particular a reactive group of the compound. In some examples, the surface of the solid-phase substrate 10 has a first group, and the compound has a second group. The first group and the second group are a set of groups that are chemically reactive, and the compound is bound to the solid-phase substrate 10 through the set of groups. In some examples, the set of groups is selected from the amino-carboxylic acid set, amino-ester group set, amino-halogen set, amino-aldehyde group set, amino-epoxy group set, mercapto-imine group set, azide-alkynyl group set, azide-alkenyl group set, and cycloalkenyl-tetrazinyl group set. It should be understood that the two groups in the set of groups are from the surface of the solid-phase substrate 10 and the compound, respectively, and that any one in the set of groups may be from the surface of the solid-phase substrate 10 and the other may be from the compound. By way of illustration, the compound is selected from one or more of an amino compound, a carboxylate compound, succinimide or an analog thereof, pentafluorophenol ester or an analog thereof, aryl halide, maleimide or an analog thereof, an epoxy compound, an aldehyde group-containing compound, an azide group-containing compound, norbornene or an analog thereof, DBCO or an analog thereof, and tetrazine and an analog thereof.

In another implementation, the solid-phase substrate 10 has a first reactant, the first fixed sequence 21 has a second reactant, the first reactant and the second reactant are reactive, and the first fixed sequence 21 is connected to the solid-phase substrate 10 by means of the first reactant and the second reactant. In some examples, the first reactant is different from the second reactant. By way of illustration, the first reactant and the second reactant are each independently selected from an antigen and an antibody, or the first reactant and the second reactant are each independently selected from biotin and streptavidin.

In some examples, a first spacer sequence region is provided between the first fixed sequence 21 and the spatial barcode sequence 22.

In some embodiments of the present application, in a specific example, the single-stranded nucleic acid molecule may further comprise a capture sequence, which is used to capture a biomolecule to be detected. The capture sequence is located at an end of the spatial barcode sequence 22 distal from the first fixed sequence 21; in other words, the capture sequence is linked to the end of the spatial barcode sequence 22 distal from the first fixed sequence 21. In this case, the single-stranded nucleic acid molecule sequentially comprises the first fixed sequence 21, the spatial barcode sequence 22, the sequencing primer binding sequence 23, the second fixed sequence 24, and the capture sequence. The capture sequence is used to capture the biological sample to be detected. The sequence length and setting of the capture sequence may be determined according to the type and nature of the biological sample to be detected. For example, the capture sequence may be a specific capture sequence, which is a sequence designed for a particular target molecule and can specifically bind to a target sample and/or a target region of the target sample, thereby improving the accuracy and specificity of detection. For example, the capture sequence may be a polyT sequence, which is a series of consecutive thymine (T) bases, and is used to capture mRNA having a polyA tail.

In some embodiments, a cleavage site is provided in the first fixed sequence 21. Thus, after using the spatial chip to perform spatial capture on the biological sample to be detected, the cleavage site of the first fixed sequence 21 is cleaved to release the single-stranded nucleic acid molecule and the object captured thereby. Therefore, in some embodiments, the solid-phase substrate 10 is a base board capable of achieving the release of the single-stranded nucleic acid molecule under cleavable conditions.

In some examples, the cleavage site may be a photocleavable site, a chemically cleavable site, or an enzyme cleavage site, and of course, may also be any two or three of said three kinds of cleavage site. It should be understood that when the cleavage site is an enzyme cleavage site, the barcode sequence does not comprise the same sequence as the cleavage site, so as to avoid the barcode sequence being cleaved.

In one embodiment, the single-stranded nucleic acid molecule may further comprise a UMI sequence. The UMI sequence is a random base sequence, which is used for quantitative analysis of the biological sample to be detected, and can improve the sensitivity and specificity of detection. In a particular example, the single-stranded nucleic acid molecule comprises, sequentially upward from the solid-phase substrate 10, the first fixed sequence 21, the spatial barcode sequence 22, the sequencing primer binding sequence 23, the second fixed sequence 24, the UMI sequence, and the capture sequence. In some examples, the UMI sequence may be 6 to 12 bases in length.

In some possible implementations, the spatial chip 100 has two or more solid-phase substrates 10, and the solid-phase substrates 10 are fixed to a surface of a solid-phase carrier according to a preset arrangement. That is, one solid-phase carrier may be provided with one solid-phase substrate 10, or may be simultaneously provided with two or more solid-phase substrates 10. By way of illustration, the spatial chip 100 has two solid-phase substrates 10, and the two solid-phase substrates 10 are fixed on the solid-phase carrier.

In certain embodiments, the solid-phase substrate 10 is provided with one or more tags. By means of providing a tag on the solid-phase substrate 10, the configuration or arrangement direction of the solid-phase substrate 10 on the surface of the solid-phase carrier, and the positional information of each of the single-stranded nucleic acid amplification clusters 20 in the solid-phase substrate 10, can be acquired. The positional information includes the physical coordinates of the single-stranded nucleic acid amplification clusters 20 and the barcode sequence of each of the single-stranded nucleic acid amplification clusters 20. Thus, when the spatial chip is used to perform spatial sample analysis, according to the barcode sequence, the spatial information of the bound sample in the original sample can be determined by means of the physical coordinates corresponding to the barcode sequence.

In examples of the present application, the manner in which the tag is provided is not strictly limited. In some embodiments, one or a combination of a letter, a number, and a graphic may be used. By way of illustration, a tag of an individual letter, a tag of an individual number, or a tag of an individual graphic may be used, or a tag containing both a letter and a number, a tag containing both a letter and a graphic, or a tag containing both a number and a graphic may be used, or a tag containing all of a letter, a number and a graphics may be used.

In examples of the present application, when the solid-phase substrate 10 is provided with multiple tags that represent different parameters, the multiple tags may be implemented using different tag graphics. For example, different letters/numbers (including different letters and/or upper- and lower-case letters, such as A, B, C, D, a, b, c, d, etc.), different patterns such as circles, triangles, rectangles, squares, ellipses or other polygons, or different letters and patterns, etc. can be used, respectively. Of course, differentiation can also be achieved by means of arranging the tag images on the solid-phase substrate 10 at different angles relative to one direction (e.g., the orientation of the apex angle of a non-equilateral triangle, etc.), or differentiation can further be achieved by using different numbers of tag graphics (e.g., one square, triangle or circle, two squares, triangles or circles arranged according to a certain arrangement rule, etc.).

In examples of the present application, a certain space is reserved between an edge of the solid-phase substrate 10 and the solid-phase carrier. This can avoid the edge of the solid-phase substrate 10 being worn or damaged during preparation, or storage and transportation, so as to avoid the loss of positional data. In some embodiments, the distance between the edge of the solid-phase substrate 10 and the edge of the solid-phase carrier is greater than or equal to 1 mm. By way of illustration, the distance between the edge of the solid-phase substrate 10 and the edge of the solid-phase carrier is, without limitation, greater than or equal to 1 mm, 2 mm, 3 mm, 4 mm, 5 mm, 6 mm, etc. In some examples, the distance between the edge of the adjacent solid-phase substrate 10 and the edge of the solid-phase carrier is one fourth or more of the length of the solid-phase substrate 10 in the direction of the distance.

In examples of the present application, the number and arrangement density of the solid-phase substrates 10 on the solid-phase carrier may be determined depending on actual need. In some examples, the distance between adjacent solid-phase substrates 10 is greater than or equal to 1 mm. By setting a suitable distance between adjacent solid-phase substrates 10, mutual interference on detection reactions can be avoided when performing the reactions, and after the reactions, detection crosstalk can be avoided when performing signal acquisition or detection on the solid-phase substrates 10.

It should be understood that when two or more solid-phase substrates 10 are arranged on one solid-phase carrier at the same time, the plurality of solid-phase substrates 10 may be arranged in an array on the surface of the solid-phase carrier. In some examples, each solid-phase substrate 10 has known positional information relative to the solid-phase carrier. The positional information is acquired by means of the chip serial number tag of the solid-phase substrate 10.

In some embodiments, an adhesive layer is provided between the solid-phase base board 11 and the solid-phase carrier, and the solid-phase substrate 10 is fixed by the bonding action of the adhesive layer. In some examples, the adhesive layer may cover the entire solid-phase base board 11 to completely bond the solid-phase base board 11 to the surface of the solid-phase carrier. In some other examples, the adhesive layer may be provided at a partial region of the solid-phase base board 11, such that the solid-phase base board 11 is partially bonded to the surface of the solid-phase carrier. It should be understood that, insofar as the same bonding material and bonding method are used, the larger the bonding region, the higher the bonding firmness between the solid-phase base board 11 and the solid-phase carrier. When the adhesive layer is provided at a partial region of the solid-phase base board 11, the region where the adhesive layer is provided may be an edge region, or may be several bonding regions uniformly or non-uniformly arranged on the adhesive layer.

In some examples, a material having a light transmittance of greater than or equal to 90%, such as an optical adhesive, is selected for the adhesive layer. Thus, the solid-phase substrate 10 obtained can be used for optical detection and will not be affected by the light transmittance of the adhesive layer. By way of illustration, the adhesive layer is selected from at least one of a transparent double-sided adhesive, an epoxy adhesive such as an epoxy resin, and an acrylate adhesive such as an acrylic resin.

The described spatial chip 100 provided by examples of the present application can be implemented in various ways. For example, the implementation can be achieved by fixing a solid-phase substrates 10 that already has complete function information onto the surface of the solid-phase carrier. Alternatively, the solid-phase substrate 10 bound to the solid-phase carrier can be treated so as to form, on the solid-phase substrate 10, sites having first positional information, and single-stranded nucleic acid molecules having a unique barcode sequence on each site.

In some embodiments, the solid-phase carrier is a solid-phase support for supporting the solid-phase substrates 10, and generally has a flat surface to achieve a flat distribution of the respective solid-phase substrates 10 on the surface thereof. The material of the solid-phase carrier is not strictly limited, and a material generally used as a base board or carrier may be used. The material may be an inorganic material such as silica, silicon nitride, silicon, quartz, glass, or ceramic. Alternatively, an organic material such as a polymer base board PET (polyethylene terephthalate) or PMMA (polymethyl methacrylate) may be used.

In some embodiments, the solid-phase carrier is selected from solid-phase carriers having a light transmittance of greater than or equal to 90%, such as transparent glass, etc. Thus, when the spatial chip 10 obtained is used for optical detection, interference of the solid-phase carrier on the detection signals can be avoided.

In some embodiments, the solid-phase carrier has a thickness of from 0.5 mm to 1.5 mm. By way of illustration, the thickness of the solid-phase carrier may be 0.5 mm ± 0.05 mm, 0.6 mm ± 0.05 mm, 0.7 mm ± 0.05 mm, 0.8 mm ± 0.05 mm, 0.9 mm ± 0.05 mm, 1.0 mm ± 0.05 mm, 1.1 mm ± 0.05 mm, 1.2 mm ± 0.05 mm, 1.3 mm ± 0.05 mm, 1.4 mm ± 0.05 mm, 1.5 mm ± 0.05 mm, etc. The thickness of the solid-phase carrier being within those ranges facilitates fixing the spatial chip in the carrier, and at the same time, facilitates optical focusing and optical imaging when performing optical signal acquisition on the spatial chip 10.

The shape of the solid-phase carrier is not strictly limited. In a possible embodiment, the shape of the solid-phase carrier is a rectangular structure, and at least one of the four right angles of the rectangular structure is configured as a chamfered corner.

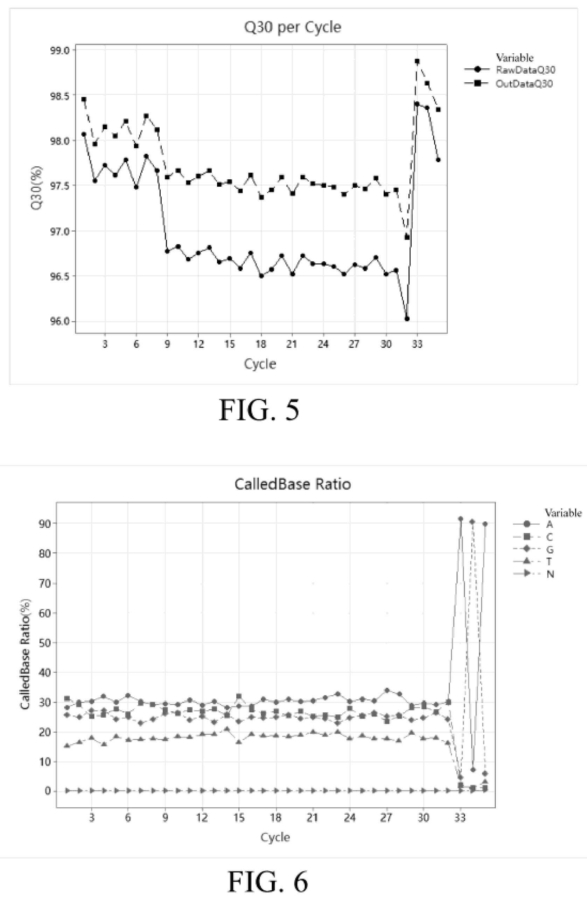

The chamfered corner in examples of the present application may be, but is not limited to, a beveled corner and/or a rounded corner. The beveled corner is sometimes also referred to as a bevel. The rounded corner is sometimes also referred to as a round corner or round chamfered corner. As shown in FIG. 5, two adjacent sides of the solid-phase carrier are connected using a line segment to replace the original right angle, thereby forming a beveled corner. It should be understood that the number of line segments forming the beveled corner may be one, or two or more. When multiple line segments are included to form the beveled corner, the multiple line segments are connected sequentially to form a regular or irregular line, and the two ends of the regular or irregular line are each connected to the respective endpoints of two adjacent sides of the rectangular base board where the chamfered corner is located. The rounded corner is formed by using an arc to connect the respective endpoints of two adjacent sides of the rectangular base board to form a chamfered corner to replace the original right angle. It should be understood that the number of arcs forming the rounded corner may be one, or two or more. When multiple arcs are included to form the rounded corner, the multiple arcs are connected sequentially to form a regular or irregular curve, and the two ends of the regular or irregular curve are each connected to the respective endpoints of two adjacent sides of the rectangular base board where the chamfered corner is located.

In examples of the present application, each of the single-stranded nucleic acid amplification clusters 20 in the solid-phase substrate 10 has known physical coordinates. When the spatial chip has two or more solid-phase substrates 10, each of the solid-phase substrates 10 has known positional information relative to the spatial chip, such that each of the single-stranded nucleic acid amplification clusters 20 on each of the solid-phase substrates 10 has known positional information relative to the spatial chip.

The method for preparing the spatial chip provided by examples of the present application is not strictly limited. As a possible implementation, the spatial chip is prepared by using the following method.

A second aspect of the present application provides a method for preparing a spatial chip (hereinafter sometimes referred to, for short, as "preparation method").

FIG. 2 is a schematic diagram of the method for preparing a spatial chip according to the present application. As shown in FIG. 2, the preparation method comprises:
(1) Hybridization, whereby single-stranded nucleic acid molecules are attached to the surface of the solid-phase substrate 10.

In this step, the single-stranded nucleic acid molecules comprise a first fixed sequence 21, a spatial barcode sequence 22, a sequencing primer binding sequence 23 and a second fixed sequence 24, and the first fixed sequence 21 and the second fixed sequence 24 are located at two ends of the single-stranded nucleic acid molecules, respectively. The structure and selection of the single-stranded nucleic acid molecules are as described above, and will not be repeated here, for the sake of brevity.

In some examples, as shown in FIG. 2, the single-stranded nucleic acid molecules sequentially comprise the first fixed sequence 21, the spatial barcode sequence 22, the sequencing primer binding sequence 23, and the second fixed sequence 24. The first fixed sequence 21, the sequencing primer binding sequence 23, and the second fixed sequence 24 all have a definite sequence. The specific sequence thereof may not be strictly specified, and the length may generally be 20 bp to 50 bp. However, the sequences of the first fixed sequence 21, the sequencing primer binding sequence 23 and the second fixed sequence 24 are different from each other, and hybridization and complementarity cannot occur between any two of them.

In the single-stranded nucleic acid molecules provided by examples of the present application, the spatial barcode sequence 22 is a unique sequence composed of a number N of nucleotides, and used for distinguishing from other different single-stranded nucleic acid molecules in the spatial chip. The value of N is related to the number of the types of the single-stranded nucleic acid molecules bound to the surface of the spatial chip. Specifically, in a spatial chip having identifiable regions, the number of the sequences formed by the possible combinations of the N nucleotides should be greater than or equal to the number of types of single-stranded nucleic acid molecules bound to the surface of the spatial chip. Since each kind of single-stranded nucleic acid molecule or single-stranded nucleic acid molecule cluster in the spatial sequences has a unique barcode sequence, the sequence and sequence position thereof can form a one-to-one correspondence on the surface of the solid-phase base board. When the spatial chip is used for spatial omics analysis, spatial information of a biological sample bound to the spatial chip may be acquired by analyzing the barcode sequence 22 and the positional information thereof.

In one particular example, the particular sequence of a single-stranded nucleic acid may be 5'-AACCGAAAAGACTATATTGCAAGGTGGATGACGGCTGGTCT ACACTGGACTGCGGAATTCTATGACGCCAGACATGTTGCGAACNNNNNNNNNNNNNNN NNNNNNNNNNNNNNNNNAGACTGAAAGTACGTGCATTACATG-3' (SEQ ID NO. 1). In the foregoing sequence, the sequence of the second fixed sequence 24 is AACGAAAGTACTTATTGCAGGTGGTACGGCTGGTCTACA (SEQ ID NO. 2), the sequence of the sequencing primer binding sequence 23 is CTGGACTGCGGAATTCTATGACGCCAGACATGTTGCGAAC (SEQ ID NO. 3), the sequence of the spatial barcode sequence 22 is 32 N's, and the sequence of the first fixed sequence 21 is AGACTGAAAGTACGTGCATTACATG (SEQ ID NO. 4).

This step further comprises providing, before hybridization, the solid-phase substrate 10 with a first probe attached to the surface thereof.

In examples of the present application, the first probe comprises a complementary sequence of the first fixed sequence 21 and a complementary sequence of the second fixed sequence 24.

In one implementation, the first probe is attached to the solid-phase substrate 10 through a linkage between groups. In some examples, the surface of the solid-phase substrate 10 has Group A, one end of the first probe has Group B, and Group A and Group B are a set of groups that are chemically reactive. In some examples, the set of groups consisting of Group A and Group B may be selected from the amino-carboxylic acid set, amino-ester group set, amino-halogen set, amino-aldehyde group set, amino-epoxy group set, mercapto-imine group set, azide-alkynyl group set, azide-alkenyl group set, and cycloalkenyl-tetrazinyl group set. It should be understood that any group in the above set of groups may be Group A, and the other group is correspondingly Group B. In some examples, the surface of the solid-phase substrate 10 is bound with a compound, and the compound has Group A. By way of illustration, the compound is selected from one or more of an amino compound, a carboxylate compound, succinimide or an analog thereof, pentafluorophenol ester or an analog thereof, aryl halide, maleimide or an analog thereof, an epoxy compound, an aldehyde group-containing compound, an azide group-containing compound, norbornene or an analog thereof, DBCO or an analog thereof, and tetrazine and an analog thereof.

In another implementation, the first probe is attached to the solid-phase substrate 10 by means of an intermolecular linkage. The solid-phase substrate 10 has a Reactant A, the first probe has a Reactant B, Reactant A and Reactant B are reactive with each other, and the first probe is attached to the solid-phase substrate 10 by means of Reactant A and Reactant B. By way of illustration, Reactant A and Reactant B are each independently selected from an antigen and an antibody, or Reactant A and Reactant B are each independently selected from biotin and streptavidin.

In some examples, the 5' end of the first probe is attached to the solid-phase substrate 10, and the first probe comprises, sequentially from the 5' end to the 3' end, the complementary sequence of the first fixed sequence 21 and the complementary sequence of the second fixed sequence 24. Thus, as shown in FIG. 2, the first fixed sequence 21 and the second fixed sequence 24 of a single-stranded nucleic acid molecule can be hybridized with the first probe through the complementary sequence of the first fixed sequence 21 and the complementary sequence of the second fixed sequence 24, respectively.

In one example, the first probe is provided with a first cleavable site. The first cleavable site is used to cleave rolling circle amplification products and the first probe off the surface of the solid-phase substrate 10 in a subsequent step. The first cleavable site is at least one of a photocleavable site, a chemically cleavable site, and an enzyme cleavage site.

In a particular example, the sequence of the first probe is: 5'-DBCO-TTTTTTTTTT-PC-link-ACCACCTGCAATAAGTACTTTCGTTCATGTAATGCACGTACTTTCAGTCT-3' (SEQ ID NO. 5), wherein the PC-link cleavable site can be cleaved after UV irradiation.

As shown in FIG. 2, the single-stranded nucleic acid molecule is hybridized with the first probe. Specifically, the first fixed sequence 21 is hybridized with the complementary sequence of the first fixed sequence 21 on the first probe, and the second fixed sequence 24 is hybridized with the complementary sequence of the second fixed sequence 24 on the first probe, such that the single-stranded nucleic acid molecule is hybridized onto the first probe, and the single-stranded nucleic acid molecule after hybridization is in a ring shape with an opening.

(2) Ring closing. The single-stranded nucleic acid molecule hybridized to the first probe is ring-closed using a ligase. That is, the first fixed sequence 21 and the second fixed sequence 24 of the single-stranded nucleic acid molecule are linked by the ligase, such that the single-stranded nucleic acid molecule forms a circular nucleic acid molecule, which can be used as a template for subsequent rolling circle amplification.

In a particular example, the first fixed sequence 21 and the second fixed sequence 24 may be linked by T4 DNA ligase to achieve ring closing of the single-stranded nucleic acid molecule.

(3) Rolling circle amplification. The circular nucleic acid molecule is subjected to rolling circle amplification using the complementary sequence of the second fixed sequence 24 as a primer to obtain a rolling circle amplification product.

Rolling circle amplification (RCA) is a nucleic acid amplification technique based on a circular DNA template. As shown in FIG. 2, a circular single-stranded nucleic acid molecule is used as a template, and the first probe, specifically the complementary sequence of the second fixed sequence in the first probe, is used as a primer. Complementary strands can be continuously synthesized by the action of a DNA polymerase, to produce an amplification product having a plurality of repeat units. Each unit is a complementary sequence of the single-stranded nucleic acid molecule, and the plurality of repeat units are cyclically repeated in the direction from the 5' end to the 3' end. Thus, highly efficient amplification of the single-stranded nucleic acid molecule is achieved. In a particular example, the polymerase used in the rolling circle amplification is Phi29DNA polymerase.

In a particular example, the rolling circle amplification is isothermal amplification, whereby amplification may be performed at a constant temperature.

(4) Sequencing. The rolling circle amplification product is sequenced using a sequencing primer to obtain sequence information of the spatial barcode sequence 22.

In this step, the sequencing primer is capable of at least partially hybridizing with a plurality of sequencing primer binding sequences 23 in the amplification product, to perform synchronous sequencing of the spatial barcode sequences 22 in the plurality of repeat units in the rolling circle amplification product, thereby increasing the sequencing signal intensity.

In some examples, the sequencing employs single-end sequencing. The number of times of base extension reaction, a.k.a., the number of sequencing cycles involved in sequencing is determined by the number of nucleotides that make up the spatial barcode sequence 22.

(5) Synthesis of a single-stranded nucleic acid molecule library. Single-stranded nucleic acid molecules are synthesized by using the rolling circle amplification product as a template to obtain a single-stranded nucleic acid molecule library.

In this step, since the rolling circle amplification product comprises complementary sequences of a plurality of single-stranded nucleic acid molecules at the same time, in the step of synthesizing a single-stranded nucleic acid molecule library, a plurality of synthesis primers are bound to the primer binding sites in the rolling circle amplification product, respectively, to simultaneously synthesize the plurality of single-stranded nucleic acid molecules. This increases the speed of synthesis of the single-stranded nucleic acid molecules, and a single-stranded nucleic acid molecule library is obtained.

In some examples, the synthesis primers are capable of hybridizing to at least a portion of the complementary sequence of the first fixed sequence or the complementary sequence of the second fixed sequence in the rolling circle amplification product to initiate the synthesis of the single-stranded nucleic acid molecule library, affording an amplification library comprising a plurality of single-stranded nucleic acid molecules. In other words, the sequence of the synthesis primers may be designed according to the sequence of the first fixed sequence 21 or the second fixed sequence 24.

In some examples, the synthesis primers are provided with a second cleavable site. By way of illustration, the second cleavable site is at least one of a photocleavable site, a chemically cleavable site, and an enzyme cleavage site. The first cleavable site and the second cleavable site are not completely identical. Thus, this enables, after the single-stranded nucleic acid molecule library is attached to the solid-phase substrate 10, subsequently detaching the single-stranded nucleic acid molecules and the conjugates thereof from the solid-phase substrate 10 by using the second cleavable site.

In a particular example, the particular sequence of the synthesis primers may be: 5'-DBCO-TTTTTTTTTT-U-CATGTAATGCACGTACTTTCAGTCT-3' (SEQ ID NO. 6), wherein U is a user enzyme cleavable site.

(6) Fixing and clustering. The single-stranded nucleic acid molecule library are fixed on the surface of the solid-phase substrate 10 to form a single-stranded nucleic acid amplification cluster 20.

In examples of the present application, the single-stranded nucleic acid molecule library synthesized in step (5) is bound to the surface of the solid-phase substrate 10 to form a single-stranded nucleic acid molecule cluster. In some examples, the 5' end of the synthesis primers is provided with a coupling group for binding to the surface of the solid-phase substrate. For example, the 5' end of the synthesis primers has a DBCO (dibenzocyclooctyne) modification, and the synthesized single-stranded nucleic acid molecule library can be bound to the solid-phase substrate 10 having an azide modification on the surface thereof.

In some examples, the surface of the solid-phase substrate 10 has a second probe, and the 5' end of the synthesis primer has a complementary sequence of the second probe. Hybridization between the second probe and the complementary sequence of the second probe enables the synthesized single-stranded nucleic acid molecule library to bind to the surface of the solid-phase substrate 10.

In a particular example, the complementary sequence of the second probe is polyN, the N being selected from one of A or a derivative thereof, T/U or a derivative thereof, G or a derivative thereof, and C or a derivative thereof.

In a particular example, after the step of fixing and clustering, the method further comprises: cleaving the rolling circle amplification product and the first probe from the surface of the solid-phase substrate 10. Specifically, the PC-link cleavable site contained in the first probe may be used. After irradiating the chip with UV, the PC-link of the first probe is cleaved, thus cleaving the rolling circle amplification product and the first probe from the surface of the solid-phase substrate 10. Thus, the chip containing the single-stranded nucleic acid molecule library on the surface of the solid-phase substrate 10 is obtained.

In a particular example, after the step of fixing and clustering, a capture sequence can further be designed according to different application scenarios and the property of a target molecule, and the capture sequence is linked to the end of the single-stranded nucleic acid molecules distal from the surface of the solid-phase substrate 10. For example, the capture sequence may be a specific capture sequence, which is a sequence designed for a particular target molecule and capable of specifically binding to the target molecule. For example, the capture sequence may be a polyT sequence, which is a series of consecutive thymine (T) bases, and is used to capture mRNA with a polyA tail.

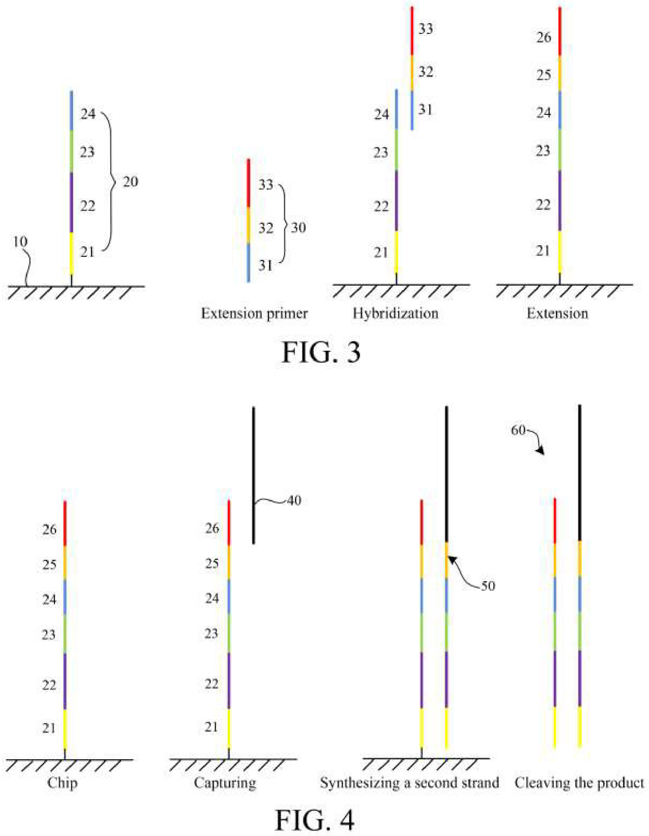

FIG. 3 is a schematic diagram of rendering a spatial chip to be linked with a capture sequence according to the present application. As shown in FIG. 3, in a particular example, an extension primer 30 can be used to render one end of a single-stranded nucleic acid molecule on the chip to be linked with a capture sequence 26. The capture sequence 26 is used to capture a biological sample to be detected. The sequence length and setting of the capture sequence 26 may be determined according to the type and nature of the biological sample to be detected. For example, the capture sequence 26 may be a specific capture sequence, which is a sequence designed for a particular target molecule and can specifically bind to a target sample and/or a target region of the target sample, thereby improving the accuracy and specificity of detection. For example, the capture sequence 26 may be a polyT sequence, which is a series of consecutive thymine (T) bases and is used to capture mRNA with a polyA tail.

In a particular example, the extension primer 30 may comprise a complementary sequence of the second fixed sequence 31 and a complementary sequence of the capture sequence 33. One end of the single-stranded nucleic acid molecule is rendered to be linked with the capture sequence 26 by means of causing the extension primer 30 and the single-stranded nucleic acid molecule on the chip to hybridize and extend. Specifically, the following manner may be adopted. The single-stranded nucleic acid molecule comprises, sequentially from the 5' end to the 3' end, the first fixed sequence 21, the spatial barcode sequence 22, the sequencing primer binding sequence 23 and the second fixed sequence 24, and the extension primer 30 comprises, sequentially from the 3' end to the 5' end, the complementary sequence of the second fixed sequence 31 and the complementary sequence of the capture sequence 33. The second fixed sequence 24 is hybridized to the complementary sequence of the second fixed sequence 31, and is used as a primer to perform extension, such that the single-stranded nucleic acid molecule comprises, sequentially from the 5' end to the 3' end, the first fixed sequence 21, the spatial barcode sequence 22, the sequencing primer binding sequence 23, the second fixed sequence 24 and the capture sequence 26.

In a particular example, the extension primer 30 may comprise the complementary sequence of the second fixed sequence 31, a complementary sequence of UMI 32 and the complementary sequence of the capture sequence 33. Thus, using the extension primer 30, the single-stranded nucleic acid molecule can be provided with the UMI sequence 25 and the capture sequence 26. The UMI sequence 25 can be used to quantify the biomolecule to be detected.

In a particular example, the sequence of the extension primer 30 may be 5'-NB AAAAAAAAAAAAAAAAAAAAAAAAAAAAAAAAAAAAAAAAAAANNNNNNNNNNAA CGAAAGTACTTATTGCAGGTGGTACGGCTGGTCTACA-3' (SEQ ID NO. 7), wherein the 5' end is a polyA sequence, and the 10 N's are a UMI sequence.

A third aspect of the present application provides a spatial transcriptomic sequencing method, comprising: performing spatial transcriptomic sequencing by using the spatial chip according to the first aspect, or the spatial chip prepared by the preparation method according to the second aspect.

FIG. 4 shows a schematic diagram of the spatial reverse transcription sequencing method according to the present application. As shown in FIG. 4, the spatial transcriptomic sequencing method specifically comprises the following:
(1) A biomolecule sample to be detected is contacted with a spatial chip such that the capture sequence 26 of the single-stranded nucleic acid molecules in the spatial chip captures the biomolecule to be detected 40. The biomolecule to be detected 40 has a sequence capable of specific complementary pairing with the capture sequence 26, and is thus captured by the capture sequence 26 to the surface of the chip.
(2) The biomolecule to be detected is extended or reverse transcribed to obtain a second strand 50, the second strand comprising the respective complementary sequences of the first fixed sequence 21, the spatial barcode sequence 22, the sequencing primer binding sequence 23, the second fixed sequence 24, and the UMI sequence 25 (if any), and the sequence of the biomolecule to be detected. Now, the surface of the spatial chip carries a double strand formed by the single-stranded nucleic acid molecule and the second strand.
(3) The formed double strand of the single-stranded nucleic acid molecule and the second strand is cleaved from the solid-phase substrate of the spatial chip using the second cleavable site to obtain a cleavage product 60.
(4) The cleavage product 60 is sequenced. The sequencing method may not be limited. Conventional platforms and reagents may be used for sequencing, so long as the sequence of the cleavage product 60 can be determined.
(5) The sequencing data obtained are analyzed. This comprises: based on the sequencing data, acquiring positional information, in the spatial chip, of the spatial barcode sequence 22 in the cleavage product 60; and based on the positional information, performing spatial restoration on the cleavage product 60, to achieve spatial reverse transcription analysis.

A fourth aspect of the present application provides a use of the spatial chip in obtaining spatial distribution information of biomolecules. The use includes spatial transcriptomic analysis, spatial proteomic analysis, and single cell spatial analysis.

The present application will be further described in detail below by way of the following particular examples. The following examples are only intended to further illustrate the present application, and should not be construed as a limitation to the present application. In the examples, unless otherwise specified, the reagents and instruments used are all conventionally commercially available, and the experimental operations are all performed according to the product manuals and conventional experimental specifications.

### Example 1

A spatial chip having positional information was prepared by means of the following method:
1) A solid-phase substrate was provided. A substrate probe having a PC-link cleavable site was coupled onto the surface of the solid-phase substrate. The PC-link cleavable site could be cleaved after UV irradiation. The particular sequence of the substrate probe was:
5'DBCO-TTTTTTTTTT-PClink-ACCACCTGCAATAAGTACTTTCGTTCATGTAATGCACGTACTTTCAGTCT-3 (SEQ ID NO. 5).
2) A single chain library was designed and synthesized. The sequence was as follows:
5'-AACGAAAGTACTTATTGCAGGTGGTACGGCTGGTCTACACTGGACTGCGGAATTCTATG ACGCCAGACATGTTGCGAACNNNNNNNNNNNNNNNNNNNNNNNNNNNNNNNNAGACT GAAAGTACGTGCATTACATG-3' (SEQ ID NO. 1), wherein the NNNNNNNNNNNNNNNNNNNNNNNNNNNNNNNNN was a spatial barcode sequence.
3) The single chain library of step 2) was introduced onto the surface of the solid-phase substrate prepared in step 1), and allowed to hybridize at 50°C for 20 min, such that the single chain library was hybridized to the surface of the solid-phase substrate through the substrate probe, thereby obtaining a chip.
4) Ring closing: The T4 DNA ligase system shown in Table 1 below was introduced into the chip and allowed to react at 37°C for 1 h, such that the T4 DNA ligase system was in contact with the single chain library hybridized on the surface of the chip and a ring closing reaction occurred, thereby obtaining circular polynucleotide templates.

**Table 1**

| Component | Volume (µL) |
|---|---|
| 10 × Ligase Buffer | 10 |
| Hieff^{®} Gold T4 DNA Ligase (5 U/µL) | 5 |
| 50% PEG20000 | 10 |
| Nuclease-free H₂O | 75 |
| Total | 100 |

5) Rolling circle amplification: The Phi29DNA polymerase amplification system shown in Table 2 below was introduced into the chip and incubated at 37°C for 1 h:

**Table 2**

| Component | Volume (µL) |
|---|---|
| 10×phi29 Reaction Buffer | 10 |
| dNTP Mix (10 mM each) | 20 |
| phi29 DNA Polymerase (10 U/µL) | 10 |
| SSB (5 mg/µL) | 10 |
| Nuclease-free H₂O | 50 |
| Total | 100 |

6) Sequencing: The chip after rolling circle amplification was subjected to sequencing using a sequencing kit. The sequencing results are as shown in FIG. 5 and FIG. 6. FIG. 5 is a diagram of the sequencing Q30 result of performing sequencing on the rolling circle amplification product according to examples of the present application, wherein the abscissa is the number of sequencing cycles, and the ordinate is the Q30 content by percent, that is, the content with a correct base identification rate of 99.9%. FIG. 5 shows that the sequencing quality Q30 of each cycle was > 90%, indicating that the sequencing quality was good. FIG. 6 is a diagram of the base distribution result of sequencing a rolling circle amplification product according to examples of the present application, wherein the abscissa represents the number of cycles of sequencing, and the ordinate represents called base ratio. According to the library design, cycles 1-32 involved random sequences, and cycles 33-35 involved a TCT fixed sequence. In the sequencing results in FIG. 6, cycles 1-32 represented random sequences, and cycles 33-35 represented an AGA sequence. Hence, the sequencing results showed that the base distribution conformed to the library design.
7) Removal of the sequencing strand: Formamide was introduced into the chip after sequencing was completed, and the sequencing primer extension strand was removed by washing.
8) Hybridization with an extension primer: The reaction was carried out at a temperature of 60°C for 10 min using 3 × SSC, wherein the sequence of the probe extension primer was:
5'-DBCO-TTTTTTTTTT-U-CATGTAATGCACGTACTTTCAGTCT-3' (SEQ ID NO. 6), wherein U is a user enzyme cleavable site.
9) On a sequencing platform, 111 cycles of extension reaction were performed to obtain a probe strand;
10) Under light-shielded conditions, the chip was irradiated with a UV lamp for 10 min, and the surface of the chip was washed with 1×SSC to detach the circular polynucleotide template, leaving only the coupled probe extension product strand.
11) Calculation of the number of probe molecules: (1) the chip was washed with 100 mM KOH such that only effective probes (including four channels of Lane01, Lane02, Lane03, and Lane04) were retained on the surface, (2) the USER enzyme digestion reaction system was introduced into the chip to react at 37°C for 30 min, (3) the liquid in the chip after USER treatment was recovered, (4) the concentration of the recovered probe was determined using a single-stranded DNA concentration assay kit, and the number of moles of the probe on each cluster was calculated, (5) the number of probe molecules (i.e., copy number) was calculated according to Avogadro's formula N = n * Na, where N represents the number of particles of a substance, n represents the number of moles of the substance, and Na represents Avogadro's constant. Calculation was performed based on the number of clusters in an area of the chip of 318 mm². It is known that the molecular weight of the probe was 44,880.00, and Avogadro's constant is 6.02×10²³. The results are shown in Table 4 below. It can be seen that the copy number of the nucleic acid molecule on the surface of the chip prepared in examples of the present application was relatively high, and was greater than 300,000/mm² for all channels.

**Table 4**

| Channel | Lane01 | Lane02 | Lane03 | Lane04 |
|---|---|---|---|---|
| Total amount of the probe recovered ng | 652.50 | 660.60 | 683.10 | 721.80 |
| Concentration of the probe recovered µM | 0.40 | 0.41 | 0.42 | 0.45 |
| Number of clusters of the probe (clusters/mm²) | 341,000 | 343,000 | 343,000 | 366,000 |
| Number of moles of the probe mol | 1.45×10⁻¹¹ | 1.47×10⁻¹¹ | 1.52×10⁻¹¹ | 1.61×10⁻¹¹ |
| mol/cluster | 1.34×10⁻¹⁹ | 1.35×10⁻¹⁹ | 1.40×10⁻¹⁹ | 1.38×10⁻¹⁹ |
| Number of molecules/cluster | 80,713 | 81,238 | 84,005 | 83,186 |

12) The solid-phase substrate was cut according to predetermined requirements and fixed on the surface of a carrier, thus obtaining the spatial chip.

### Example 2: Preparation of an oligo dT capture chip

13) The chip in step 10) of Example 1, which has completed coupling, was washed with formamide to obtain the nucleic acid molecule probe in single-stranded form.

14) A Klenow extension system was prepared, mixed uniformly, and placed on ice for later use. The sequence of the extension primer was:
5'-NBAAAAAAAAAAAAAAAAAAAAAAAAAAAAAAAAAAAAAAAAAAANNNNNNNNNN AACGAAAGTACTTATTGCAGGTGGTACGGCTGGTCTACA-3' (SEQ ID NO. 7, the N's represent a random sequence, and B represents non-A (i.e., B represents one of G, C and T)). The Klenow extension system is as shown in Table 5 below:

**Table 5**

| Component | Volume (µL) |
|---|---|
| 10 × Blue Buffer | 10 |
| Klenow Fragment exo | 6 |
| dNTP Mix (10 mM each) | 14 |
| Extension primer (100 µM) | 10 |
| NF-H₂O | 60 |
| Total | 100 |

15) The Klenow extension system was introduced into the chip, and extension was carried out at a temperature of 37°C for 1 h.

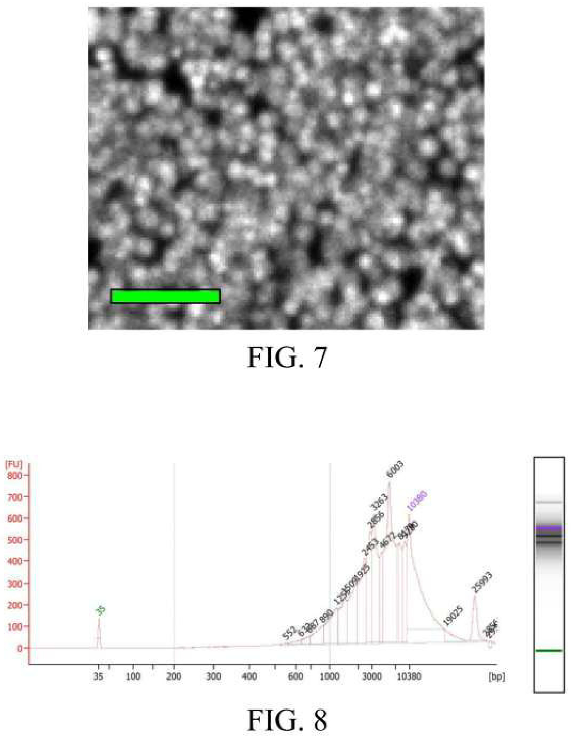

16) The chip, after the extension, was subjected to quality control using 1 µM A30 cy3 probe. FIG. 7 is a diagram of the quality control result of the quality control performed on the spatial chip according to examples of the present application. The quality control result showed fluorescent signals, indicating that the chip prepared in the example could capture RNA. That is, in the example, a single-stranded nucleic acid molecule cluster was successfully prepared according to a preset nucleic acid molecule structure, and extension of the structure was achieved on the single-stranded nucleic acid molecule cluster. Note: The bar represents a 10 µm scale.

### Example 3: Free RNA testing

1) The chip in Example 2 was reacted with 100% formamide at a temperature of 50°C, and then washed with enzyme-free water and dried for later use.
2) A FreeRNA hybridization system was prepared according to Table 6 below. The FreeRNA hybridization system was added dropwise to the chip, and hybridization was carried out at room temperature for 15 min:

**Table 6**

| Component | Volume (µL) |
|---|---|
| 20×SCC | 50 |
| Free RNA (528 ng/µL) | 4 |
| RNase inhibitor (40 u/µL) | 20 |
| Nuclease-free H₂O | 126 |
| Total | 200 |

3) A reverse transcription reaction system was prepared according to Table 7 below. After completing preparation, the reverse transcription reaction system was mixed uniformly, instantaneously centrifuged, and placed on ice for later use.

**Table 7**

| Component | Volume (µL) |
|---|---|
| 5× Maxima H RT Buffer | 20 |
| RNase inhibitor (40 u/µL) | 5 |
| Maxima H (200 U/µL) | 5 |
| dNTP Mix (10 mM each) | 10 |
| TSO (100 µM) | 5 |
| 5M Betaine | 20 |
| 50% PEG20000 | 10 |
| Nuclease-free H₂O | 25 |
| Total | 100 |

The sequence of TSO was:
5'-GAGTGGAACTGGATGGTCGCAGGTATCTCGTC/rG//rG//iXNA_G/-3' (SEQ ID NO. 8), where /rG/ represents the ribose form of guanosine, that is, rG represents a ribonucleotide (G in RNA); /rG//rG/ represents two consecutive ribonucleotides (rG); /iXNA_G/ represents an iXNA modified guanosine (G).
4) The free RNA hybridization system was removed, and the reverse transcription reaction system was added dropwise thereonto. The reaction was carried out at a temperature of 55°C for 2 h, and then at a temperature of 42°C overnight.
5) A User enzyme digestion system was prepared according to Table 8 below. After completing preparation, the User enzyme digestion system was mixed uniformly, instantaneously centrifuged, and placed on ice for later use.

**Table 8**

| Component | Volume (µL) |
|---|---|
| User (1 U/µL) | 5 |
| 10×rCutsmart | 10 |
| 50% PEG8000 | 8 |
| Nuclease-free H2O | 77 |
| total | 100 |

6) The reverse transcription reaction system was removed, and the User enzyme digestion system was added dropwise thereonto. The reaction was carried out at a temperature of 37°C for 1 h. Every 30 min, the reaction was gently pipetted once with a pipette, care being taken to avoid bubbles.
7) The User reaction system was recovered and reacted at a temperature of 75°C for 10 min to inactivate the enzyme.
8) A cDNA amplification system was prepared according to Table 9 below. After completing preparation, the cDNA amplification system was mixed uniformly, instantaneously centrifuged, and placed on ice for later use.

**Table 9**

| Component | Volume (µL) |
|---|---|
| 2×KAPA HiFi HotStart ReadyMix | 100 |
| cDNA_F (10µM) | 10 |
| cDNA_R (10µM) | 10 |
| dNTP Mix (10 mM each) | 20 |
| User reaction system recovery | 60 |
| Total | 200 |

The sequences of cDNA_F and cDNA_R were as follows, respectively:
cDNA_F: 5'-CATGTAATGCACGTACTTTCAGTCT-3' (SEQ ID NO.9); and
cDNA_R: 5'-GACGAGATACCTGCGACCATCCAGTTCCACTC-3' (SEQ ID NO.10).
9) A PCR reaction was carried out according to the amplification procedure shown in Table 10 below:

**Table 10**

| Temperature | Time | Cycle number |
|---|---|---|
| 105°C | Heat cover | 1 |
| 95°C | 5 min | |
| 98°C | 20 s | 15 |
| 60°C | 20 s | |
| 72°C | 30 s | |
| 72°C | 3 min | 1 |
| 12°C | Hold | |

10) According to the instructions for DNA magnetic bead purification, the cDNA product was recovered using 1 ×VAHTS DNA Clean Beads. The product was identified using Agilent 2100, and the diagram of distribution of the obtained cDNA fragments is as shown in FIG. 8. FIG. 8 shows that the distribution of the obtained cDNA fragments was within a reasonable fragment distribution range.
11) According to the instructions for Tn5 transposase, the following linkers were embedded:

| Primer name | Primer sequence |
|---|---|
| Primer A | 5'-phos-CTGTCTCTTATACACATCT-NH2-3' (SEQ ID NO.11) |
| Primer B | 5'-TCGTCGGCAGCGTCAGATGTGTATAAGAGACAG-3' (SEQ ID NO.12) |

12) According to the instructions for Tn5 transposase, the embedded Tn5 transposase was used to perform a fragmentation reaction on the cDNA products.
13) The fragmentation products were amplified by PCR. A system was prepared according to Table 11 below. After completing preparation, the system was mixed uniformly, instantaneously centrifuged, and placed on ice for later use.

**Table 11**

| Component | Volume (µL) |
|---|---|
| 2×KAPA HiFi HotStart ReadyMix | 50 |
| lib_F (10 µM) | 5 |
| lib_R (10 µM) | 5 |
| dNTP Mix (10 mM each) | 10 |
| Fragmentation products | 30 |
| Total | 100 |

The sequences of lib_F and lib_R were as follows, respectively:
lib_F: 5'-CATGTAATGCACGTACTTTCA*G*T*C*T-3' (SEQ ID NO. 13). The bases with the superscript (*) represent thio-modified bases. For example, G* represents thio-modified guanosine.
lib_R: 5'-TCGTCGGCAGCGTCAGATGTGTATAAGAGACAG-3' (SEQ ID NO.14).
14) PCR reaction was performed according to the amplification procedure shown in Table 12 below:

**Table 12**

| Temperature | Time | Cycle number |
|---|---|---|
| 105°C | Heat cover | 1 |
| 95°C | 5 min | |
| 98°C | 20 s | 8 |
| 60°C | 20 s | |
| 72°C | 30 s | |
| 72°C | 3 min | 1 |
| 12°C | Hold | |

15) According to the instructions of DNA magnetic bead purification, the fragmentation products after amplification were recovered by performing of 0.5× purification and two successive times of 0.15× purification.

### Example 4: Data analysis

1) The products in Example 3 were subjected to high-throughput sequencing on a Genolab M sequencing platform using the PE100 strategy to obtain sequencing data.
2) Data comparison and analysis: The 32 N and 10 UMI information of the first strand in the fq obtained by sequencing was extracted. The 32 N data were compared with the fq data of the first sequencing in Example 1 to obtain positional information. The UMI was used for quantifying the gene expression. The cDNA information of the second strand in the fq obtained by sequencing was used for gene comparison. The results are as shown in the table below. Table 13 below shows the gene capture numbers obtained by different bin sizes after comparison.

**Table 13**

| BinSize | MeanUMI | MedianUMI | MeanGeneNum | MedianGeneNum |
|---|---|---|---|---|
| bin100µm | 23533 | 24381 | 3071 | 3252 |
| bin50µm | 5977 | 6169 | 1418 | 1516 |
| bin25µm | 1510 | 1553 | 589 | 623 |
| bin20µm | 972 | 996 | 434 | 456 |
| bin10µm | 247 | 250 | 157 | 163 |

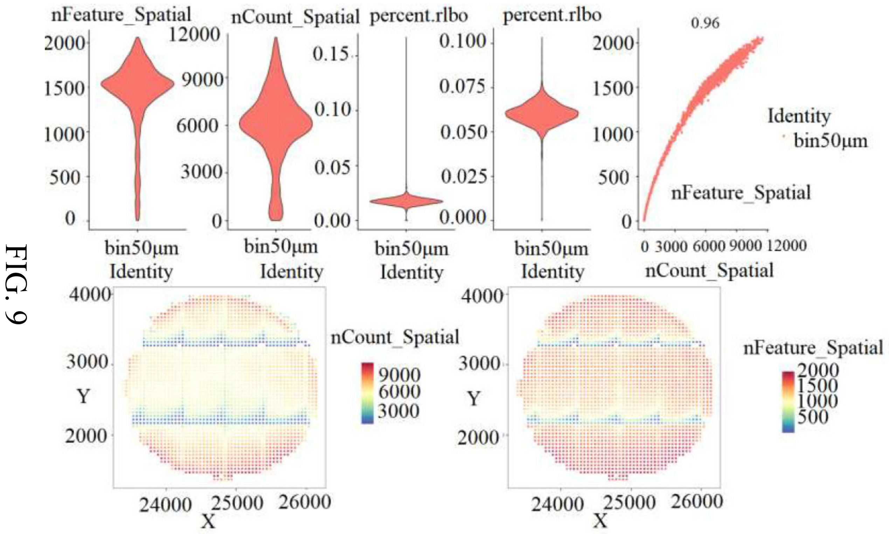

In the above table, binXµm denotes a window obtained by division and having a size of X µm × X µm, for example, bin100µm denotes a window obtained by division and having a size of 100 µm × 100 µm; MeanUMI denotes the mean number of bound UMI, MedianUMI denotes the median number of bound UMI, MeanGeneNum denotes the mean number of bound Gene types, and MedianGeneNum denotes the median number of bound Gene types.
3) The data after completion of comparison was plotted against bin50µm to confirm whether the spatial positional information was restored. The results are as shown in FIG. 9, which is a diagram of the result of restoring spatial positional information by plotting compared data against bin50µm according to examples of the present application. The ordinate of the first two panels of FIG. 9 represents number, and the two panels denote the number distribution of bound Gene and UMI, respectively; panels 3 and 4 show the graphical representation obtained by calculating the log value of the number of bound Gene types and UMI, respectively; the abscissa of panel 5 denotes the number of bound UMI and the ordinate denotes the number of bound Gene types. It can be seen that as the number of UMI increased, the identified gene types increased and eventually tended to saturate. Taking bin50µm as an example, the sequences of bound UMI in the window were detected, and restored to the window according to the positional information corresponding to the sequences, thus obtaining panel 6 of FIG. 9; the Gene types were restored to the window according to the positional information, thus obtaining panel 7 of FIG. 9. It can be seen that the spatial chip provided by examples of the present application could restore the spatial positional information and achieve Free RNA spatial transcriptomic analysis.

The above content is further detailed descriptions of the present application in conjunction with embodiments, but the specific implementation of the present application shall not be considered to be only limited to these descriptions. For those of ordinary skill in the art to which the present application belongs, several simple deductions or replacements may be made without departing from the conception of the present application.

## Claims

1. A method for preparing a spatial chip, **characterized by** comprising:
hybridizing to attach a single-stranded nucleic acid molecule to a surface of a solid-phase substrate, wherein the single-stranded nucleic acid molecule comprises a first fixed sequence, a spatial barcode sequence, a sequencing primer binding sequence and a second fixed sequence, the first fixed sequence and the second fixed sequence are located at two ends of the single-stranded nucleic acid molecule respectively, a first probe is attached to the surface of the solid-phase substrate, and the first probe comprises a complementary sequence of the first fixed sequence and a complementary sequence of the second fixed sequence;
ring closing, comprising: linking the first fixed sequence and the second fixed sequence by using a ligase, such that the single-stranded nucleic acid molecule forms a circular nucleic acid molecule;
rolling circle amplification, comprising: subjecting the circular nucleic acid molecule to rolling circle amplification by using the complementary sequence of the second fixed sequence as a primer;
sequencing, comprising: sequencing the rolling circle amplification product by using a sequencing primer, to obtain sequence information of the spatial barcode sequence;
synthesizing a single-stranded nucleic acid molecule library, comprising: synthesizing the single-stranded nucleic acid molecule by using the rolling circle amplification product as a template, to obtain a single-stranded nucleic acid molecule library; and
fixing and clustering, comprising: fixing the single-stranded nucleic acid molecule library onto the surface of the solid-phase substrate, to form a single-stranded nucleic acid molecule cluster.

2. The preparation method according to claim 1, wherein the single-stranded nucleic acid molecule comprises, sequentially from the 5' end to the 3' end, the first fixed sequence, the spatial barcode sequence, the sequencing primer binding sequence and the second fixed sequence, the first probe sequentially comprises the complementary sequence of the second fixed sequence and the complementary sequence of the first fixed sequence, and the probe is attached to the surface of the solid-phase substrate by means of the complementary sequence of the first fixed sequence.

3. The preparation method according to claim 2, wherein the first probe is provided with a first cleavable site.

4. The preparation method according to claim 3, wherein the first cleavable site is at least one of a photocleavable site, a chemically cleavable site, and an enzyme cleavage site.

5. The preparation method according to claim 3, wherein, after the step of fixing and clustering, the rolling circle amplification product and the first probe are cleaved from the surface of the solid-phase substrate by means of the first cleavable site.

6. The preparation method according to any one of claims 1-5, wherein the spatial barcode sequence is formed by polymerization of a number n of nucleotides, and the number of the single-stranded nucleic acid molecule clusters on the surface of the solid-phase substrate is less than or equal to 4ⁿ.

7. The preparation method according to any one of claims 1-5, wherein, in the step of synthesizing the single-stranded nucleic acid molecule library, a sequence in which a second cleavable site is provided is used as a synthesis primer for synthesizing the single-stranded nucleic acid molecule.

8. The preparation method according to claim 7, wherein the second cleavable site is at least one of a photocleavable site, a chemically cleavable site, and an enzyme cleavage site.

9. The preparation method according to claim 7, wherein the first probe is provided with the first cleavable site, and the first cleavable site and the second cleavable site are not completely identical.

10. The preparation method according to claim 7, wherein the 5' end of the synthesis primer is provided with a coupling group, the coupling group being used for binding to the surface of the solid-phase substrate; and/or
the surface of the solid-phase substrate is linked with a second probe, and the 5' end of the synthesis primer is provided with a complementary sequence of the second probe;
optionally, wherein the complementary sequence of the second probe is polyN, the N being selected from one of A or a derivative thereof, T/U or a derivative thereof, G or a derivative thereof, and C or a derivative thereof.

11. The preparation method according to any one of claims 1-10, wherein, after the step of fixing and clustering, the single-stranded nucleic acid molecule library is linked with a capture sequence, the capture sequence being used for capturing a biomolecule to be detected;
optionally, wherein the capture sequence comprises polyT;
optionally, wherein the single-stranded nucleic acid molecule library is linked with the capture sequence and a UMI sequence, the UMI sequence being a random base sequence.

12. The preparation method according to any one of claims 1 to 11, wherein the single-stranded nucleic acid molecule cluster has a cross-sectional diameter of 0.5 µm to 1 µm, and the copy number of the single-stranded nucleic acid molecule in the single-stranded nucleic acid molecule cluster is greater than 30,000.

13. A spatial chip prepared by the preparation method according to any one of claims 1 to 12.

14. A use of the spatial chip according to claim 15 in obtaining spatial distribution information of biomolecules.

15. The use according to claim 14, wherein the use includes spatial transcriptomic analysis, spatial proteomic analysis, and single cell spatial analysis.
